# EUROPÄISCHE PATENTSCHRIFT

(11) **EP 1 653 895 B1**
(45) Veröffentlichungstag und Bekanntmachung des Hinweises auf die Patenterteilung: **07.02.2007**
(21) Anmeldenummer: 05715894.1
(22) Anmeldetag: 10.03.2005
(51) Int. Cl.: A61F 5/41

(54) **KONDOM MIT EINER BESCHICHTUNG UND ZUSAMMENSETZUNG**
CONDOM WITH A COATING AND COMPOSITION
CONDOM AYANT UN REVETEMENT ET COMPOSITION

(30) Priorität: 28.04.2004 DE 202004006803 U
(43) Veröffentlichungstag der Anmeldung: 10.05.2006
(73) Patentinhaber: Brücker, Achim, 53225 Bonn (DE)
(72) Erfinder: Brücker, Achim, 53225 Bonn (DE)
(74) Vertreter: Müller, Thomas Michael
(86) Internationale Anmeldenummer: PCT/EP2005/002512
(87) Internationale Veröffentlichungsnummer: WO 2005/107660

(56) Entgegenhaltungen:
- WO-A-00/50507
- WO-A-02/078580
- WO-A-03/088880
- DE-A1- 3 943 519
- US-A- 4 829 991
- US-A- 5 333 621
- US-A- 5 879 682
- US-A- 5 952 361
- US-A1- 2003 130 183
- US-B1- 6 482 857

## Beschreibung

Die Erfindung betriff ein Kondom mit einer Beschichtung auf einer Innenfläche des Kondoms zur Unterstützung der Erektion bei Männern, insbesondere bei Erektionsstörungen. Ferner betrifft die Erfindung eine Zusammensetzung sowie die Verwendung einer Kombination von Verbindungen zur Bereitung einer Zusammensetzung zur lokalen Anwendung am Penis.

Erektionsstörungen sind ein weitverbreitetes Problem, zu dem eine Vielzahl von Behandlungsmöglichkeiten existieren. WO 03/088880 A2 offenbart ein Kondom mit einer vasodilatorischen Beschichtung auf einer Innenfläche des Kondoms. US 2003/0130183 A1 und US 5 879 682 offenbaren Zusammensetzungen zur genitalen Anwendung, welche als vasodilatorische Substanz u. a. Gingerol umfassen. Als weitere Behandlungsmöglichkeit wird zum Beispiel die Injektion gefäßerweiternder Zusammensetzungen in den Penis unmittelbar von dem Geschlechtsverkehr vorgeschlagen. In EP 1 049 461 B1 ist hingegen eine Verwendung einer Zusammensetzung zur lokalen Anwendung am Penis beschrieben, die Glycerintrinitrat und Lanolin enthält. Ferner wird dort beschrieben, dass diese Zusammensetzung in Verbindung mit einem Kondom angewendet werden kann. Ebenso zeigt bereits US 4 829 991 ein Kondome mit Innenbeschichtungen, die den Wirkstoff Glycerintrinitrat zur Durchblutungsförderung beinhalten.

Durch den Wirkstoff Glycerintrinitrat mit seiner durchblutungsfördernden Wirkung soll die Erektion unterstützt werden, wobei jedoch ein Blutdruckabfall auftreten kann.

Aufgabe der vorliegenden Erfindung ist es, ein Mittel bereitzustellen, das eine ver-besserte Wirkung bei der Anwendung zur Unterstützung der Erektion bei Männern aufweist.

Die Aufgabe wird erfindungsgemäß durch ein Kondom mit einer Beschichtung zur Unterstützung der Erektion bei Männern, wobei die Beschichtung auf einer Innenfläche des Kondoms aufgebracht ist und Ingwer-Wurzelextrakt als einzigen wirksamen Inhaltsstoff enthält, gelöst.

Ingwer hat bekanntermaßen eine durchblutungsfördernde Wirkung und unterstützt daher bei der Verwendung eines Kondoms mit einer Innenbeschichtung mit Ingwer-Wurzelextrakt die Erektion des Penis.

Um möglichst wenig Irritationen auszulösen, ist vorzugsweise vorgesehen, dass die Beschichtung in einem Bereich der Innenfläche des Kondoms vorgesehen ist, der bei Anwendung des Kondoms mit dem Schwellkörperbereich des Penis in Kontakt kommt. Die übrigen Bereiche der Innenfläche des Kondoms sind nicht mit der Beschichtung versehen.

Die Beschichtung kann auch auf die Außenfläche des Kondoms aufgebracht ist, um den Sexualpartner ebenfalls zu stimulieren.

Ferner wird die Aufgabe durch eine Zusammensetzung in Form einer Creme oder Salbe zur lokalen Anwendung am Penis zur Unterstützung der Erektion, wobei die Zusammensetzung Ingwer-Wurzelextrakt als einzigen wirksamen Inhaltsstoff enthält, gelöst.

Die Creme oder Salbe kann eine herkömmliche Zusammensetzung aufweisen. Beispielsweise kann eine Salbe die Bestandteile Weizenstärke, Wasser, Glycerin, Weingeist und Traganth aufweisen.

Ferner wird die Aufgabe durch die Verwendung einer Kombination von Verbindungen bei der Bereitung einer Zusammensetzung zur lokalen Anwendung am Penis zur Unterstützung der Erektion gelöst, wobei die Zusammensetzung Ingwer-Wurzelextrakt als einzigen wirksamen Inhaltsstoff enthält.

## Patentansprüche

1. Kondom mit einer Beschichtung zur Unterstützung der Erektion bei Männern, wobei die Beschichtung auf einer Innenfläche des Kondoms aufgebracht ist,
**dadurch gekennzeichnet,**
**dass** die Beschichtung Ingwer-Wurzelextrakt als einzigen wirksamen Inhaltsstoff enthält.

2. Kondom nach Anspruch 1,
**dadurch gekennzeichnet,**
**dass** die Beschichtung in einem Bereich der Innenfläche des Kondoms vorgesehen ist, der bei Anwendung des Kondoms mit dem Schwellkörperbereich des Penis in Kontakt kommt.

3. Kondom nach einem der Ansprüche 1 oder 2,
**dadurch gekennzeichnet,**
**dass** die Beschichtung auch auf die Außenfläche des Kondoms aufgebracht ist.

4. Zusammensetzung in Form einer Creme oder Salbe zur lokalen Anwendung am Penis zur Unterstützung der Erektion, wobei die Zusammensetzung Ingwer-Wurzelextrakt als einzigen wirksamen Inhaltsstoff enthält.

5. Verwendung einer Kombination von Verbindungen bei der Bereitung einer Zusammensetzung zur lokalen Anwendung am Penis zur Unterstützung der Erektion, wobei die Zusammensetzung Ingwer-Wurzelextrakt als einzigen wirksamen Inhaltsstoff enthält.

## Claims

1. A condom having a coating for supporting the erection of men, wherein the coating is applied to the inside surface of the condom,
**characterized in**
**that** ginger-root extract is the only effective ingredient contained in the coating.

2. The condom according to Claim 1,
**characterized in**
**that** the coating is provided on the inside surface of the condom in an area coming into contact with the erectile body region of the penis.

3. The condom according to Claims 1 or 2,
**characterized in**
**that** the coating is also applied to the outside surface of the condom.

4. A composition in the form of a cream or an ointment for local application on the penis for supporting an erection, wherein the composition contains ginger-root extract as the only effective ingredient.

5. Use of a combination of compounds for preparing a composition for local application on the penis for supporting an erection, wherein the composition contains ginger-root extract as the only effective ingredient.

## Revendications

1. Condom avec un revêtement pour soutenir l'érection chez les hommes, où le revêtement est appliqué sur une surface interne du condom, **caractérisé en ce que** le revêtement contient un extrait de racine de gingembre comme seul constituant efficace.

2. Condom selon la revendication 1, **caractérisé en ce que** le revêtement est prévu dans un domaine de la surface interne du condom qui vient en contact avec le domaine du corps érectile du pénis lors de l'utilisation du condom.

3. Condom selon l'une des revendications 1 ou 2, **caractérisé en ce que** le revêtement est appliqué sur la surface externe du condom.

4. Composition sous forme d'une crème ou d'une pommade pour l'utilisation locale sur le pénis pour soutenir l'érection, où la composition contient un extrait de racine de gingembre comme seul constituant efficace.

5. Utilisation d'une combinaison de composés dans la production d'une préparation pour l'utilisation locale sur le pénis pour soutenir l'érection, où la composition contient un extrait de racine de gingembre comme seul constituant efficace.
